(19) **Europäisches Patentamt**
**European Patent Office**
**Office européen des brevets**

(11) **EP 1 527 729 B1**

(12) **EUROPEAN PATENT SPECIFICATION**

(45) Date of publication and mention
of the grant of the patent:
**04.03.2009 Bulletin 2009/10**

(51) Int Cl.:
***A61B 1/04*** *(2006.01)* ***H04N 9/73*** *(2006.01)*

(21) Application number: **04025829.5**

(22) Date of filing: **29.10.2004**

(54) **Image processing apparatus**

Bildverarbeitungsvorrichtung

Dispositif de traiment d'images

(84) Designated Contracting States:
**DE FR GB**

(30) Priority: **30.10.2003 JP 2003371118**

(43) Date of publication of application:
**04.05.2005 Bulletin 2005/18**

(73) Proprietor: **Olympus Corporation
Tokyo 151-0072 (JP)**

(72) Inventors:
• **Imaizumi, Katsuichi
Hachioji-shi
Tokyo (JP)**
• **Doguchi, Nobuyuki
Hino-shi
Tokyo (JP)**
• **Takahashi, Yoshinori
Hachioji-shi
Tokyo (JP)**

• **Ozawa, Takeshi
Sagamihara-shi
Kanagawa (JP)**
• **Takehana, Sakae
Sagamihara-shi
Kanagawa (JP)**
• **Hirao, Isami
Hachioji-shi
Tokyo (JP)**

(74) Representative: **von Hellfeld, Axel
Wuesthoff & Wuesthoff
Patent- und Rechtsanwälte
Schweigerstrasse 2
81541 München (DE)**

(56) References cited:
**WO-A-01/26050       US-A- 5 058 603
US-A- 5 570 129       US-A- 5 926 213
US-B1- 6 293 911       US-B1- 6 421 083**

## Description

BACKGROUND OF THE INVENTION

1. Field of the Invention

The present invention relates to apparatuses each having an image pick-up function, e.g., electronic endoscope systems and, more particularly, to an image processing apparatus and an endoscope system each adjusting the color balance of image signals obtained by picking up an image of an object.

2. Description of the Related Art

Electronic endoscope systems capable of performing various treatments are generally used. According to the electronic endoscope system, a scope is inserted into a body cavity to observe a trachea, a lung, or a digestive tract such as an esophagus, a stomach, a small intestine, or a large intestine. As necessary, treatment is performed with instruments inserted through an instrument channel in the scope.

Diagnosis using the electronic endoscope system is generally performed based on normal observation in which a color image similar to that observed macroscopically is displayed in a monitor. Further, auto-fluorescent observation utilizing auto-fluorescence in living-body tissue is starting to be available. The auto-fluorescent observation uses the following phenomenon: Upon exposure to excitation light ranging from ultraviolet to blue, the spectrum of auto-fluorescence in living-body tissue of a tumor is different from that of a normal mucosa. In the monitor, an image formed by auto-fluorescence is displayed together with an image formed by light reflected from the living-body tissue in the corresponding different colors. Thus, a lesion can be precisely distinguished from the normal tissue (for example, Japanese Unexamined Patent Application Publication No. 2002-336196).

For example, as disclosed in Japanese Unexamined Patent Application Publication No. 2002-95635, narrow band imaging (NBI) is also available. In the narrow band imaging, light having a wavelength that is narrower than that of normal observation light is used for observation. According to the narrow band imaging, a superficial blood vessel can be observed with higher contrast.

Infrared observation with near infrared light is also used. In the infrared observation, an agent which absorbs near infrared light, e.g., indocyanine green (ICG) is infused into a blood vessel, so that blood circulation in a deep region under a mucosa can be observed. The blood circulation in the deep region cannot be observed in the normal observation.

In the auto-fluorescent observation, the narrow band imaging, and the infrared observation, disadvantageously, a variation in mucosal color depending on patient or scene is larger than that in the normal observation.

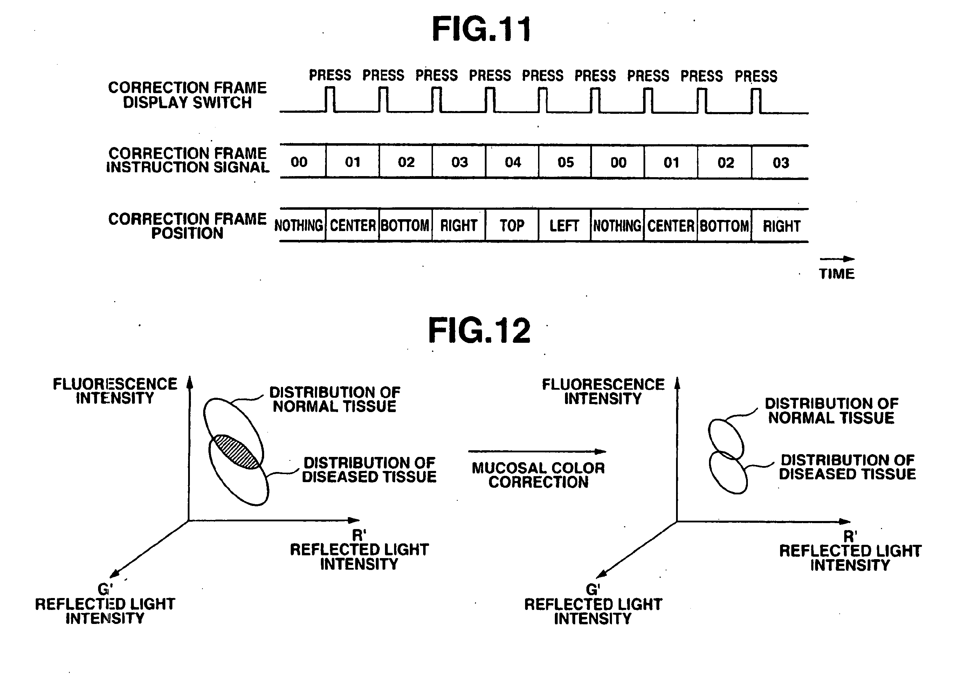

The content of fluorescent material such as collagen in a living body and the thickness of an epithelium on the fluorescent material extremely vary depending on patient. In the auto-fluorescent observation, therefore, the color of obtained fluorescent image also greatly varies depending on patient. In the auto-fluorescent observation, since the color distribution of normal tissue is different from that of diseased tissue as shown in the left of Fig. 12, a lesion is distinguished from the normal tissue on the basis of the color of an image. However, the mucosal color varies depending on patient. This leads to the increase in area (hatched portion) where the color distribution of normal tissue overlaps that of diseased tissue. Consequently, correct diagnosis may be hardly performed.

The narrow band imaging uses light of 400 to 430 nm in wavelength, in which the absorbance of blood is very high. Disadvantageously, the color of an image considerably varies depending on patient. The variation may interfere with correct diagnosis.

In the infrared observation, the color of a mucosa greatly varies before and after ICG is intravenously administered. In order to view a blood vessel with high contrast, it is necessary to adjust the color balance of an image so as to see the blood vessel most dearly. Disadvantageously, the operation is complicated.

Other examples of conventional systems for colour balance adjustment are described in US 5 570 129, US 5 926 213, US 6 421083 and US 6 293 911.

US 5,058,603 discloses that a reference color display device is used for color adjustment. The outer peripheral surface of a distal end portion is colored in three primary colors, that is, red, green and blue. The reference color display device for color tone adjustment is inserted through the forceps channel in the so-called electronic endoscope having a solid state image pick-up element The distal end portion 105 is pressed into intimate contact with the surface of the mucous membrane of the body cavity, the distal end portion colored in the three reference colors is held generally flush with the surface of the mucous membrane and the distal end portion can be observed in the vincity of the centre of the field of vision. In this condition, while observing the pick-up image displayed on a monitor of the endoscope, the color tone of the image is adjusted so that the displayed three colors conincide with their respective actual colors. Thus, under the same condition for an actual observation of the mucous membrane surface of the body cavity, the color tone adjustment can be carried out using the predetermined three primary colors as a reference.

WO 01/26050 relates to color correction of an isolated object In an image. It is proposed to use object segmentation methods before carrying out color correction.

It is an object of the present invention to provide an image processing apparatus capable of adjusting the color balance of an image of an object according to the object with simple operation.

## SUMMARY OF THE INVENTION

The present Invention provides an image processing apparatus having the features recited in claim 1. Preferred features of the invention are recited in the dependent claims.

Thus, the present invention provides an image processing apparatus including color balance adjustment means and control means. The color balance adjustment means adjusts the color balance of image signals obtained by picking up an image of an object through image pickup means. The control means controls the color balance adjustment means based on feature data that characterizes the color of the object.

According to the present invention, since the apparatus includes the control means for controlling the color balance adjustment means based on feature data characterizing the color of the object, the color of an image of an object can be properly adjusted according to the object

## BRIEF DESCRIPTION OF THE DRAWINGS

Fig. 1 is a block diagram of the entire structure of an endoscope system according to an embodiment of the present invention;
Fig. 2 is a diagram explaining a band switch filter;
Fig. 3 is a diagram explaining a rotating filter wheel;
Fig. 4 shows the transmission characteristic of a normal/fluorescent observation filter and that of an infrared observation filter;
Fig. 5 shows the transmission characteristic of a narrow band imaging (NBI) filter;
Fig. 6 shows the transmission characteristics of R, G, and B filters;
Fig. 7 shows the transmission characteristics of excitation, G', and R' filters;
Fig. 8 shows the transmission characteristic of an excitation-light cut filter;
Fig. 9 is a flowchart of a process for determining an observation mode to be supported;
Fig. 10 is a diagram explaining an observation screen;
Fig. 11 is a diagram explaining the correction frame display operation;
Fig. 12 explains a change in color distributions by mucosal color correction;
Fig. 13 is a diagram explaining a color balance level display LED; and
Fig. 14 is a diagram explaining the image recording operation.

## DESCRIPTION OF THE PREFERRED EMBODIMENTS

An embodiment of the present invention will now be described with reference to the drawings.

In the present embodiment, an image processing apparatus for performing color balance adjustment included in an electronic endoscope system (below, simply referred to as an endoscope system) will now be described.

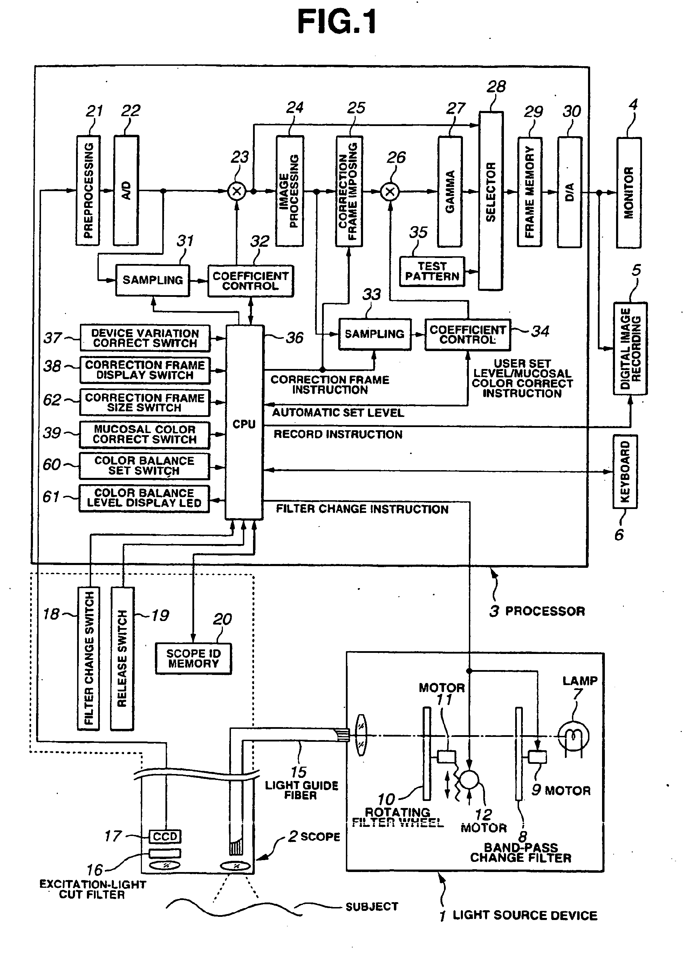

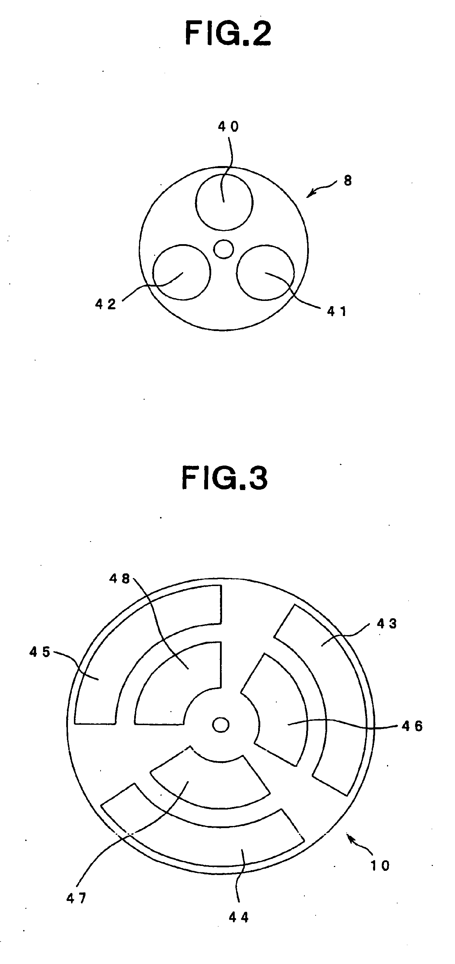

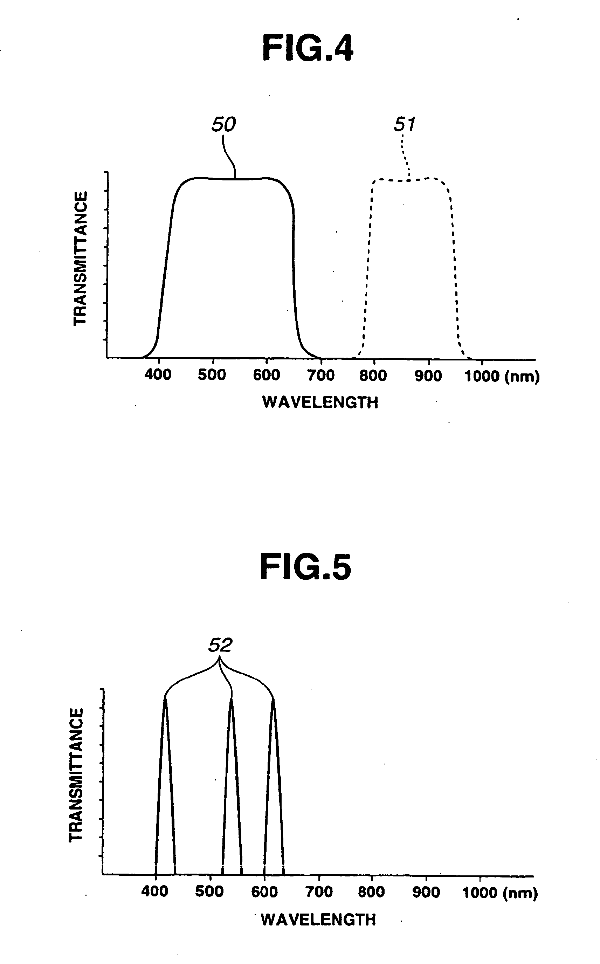

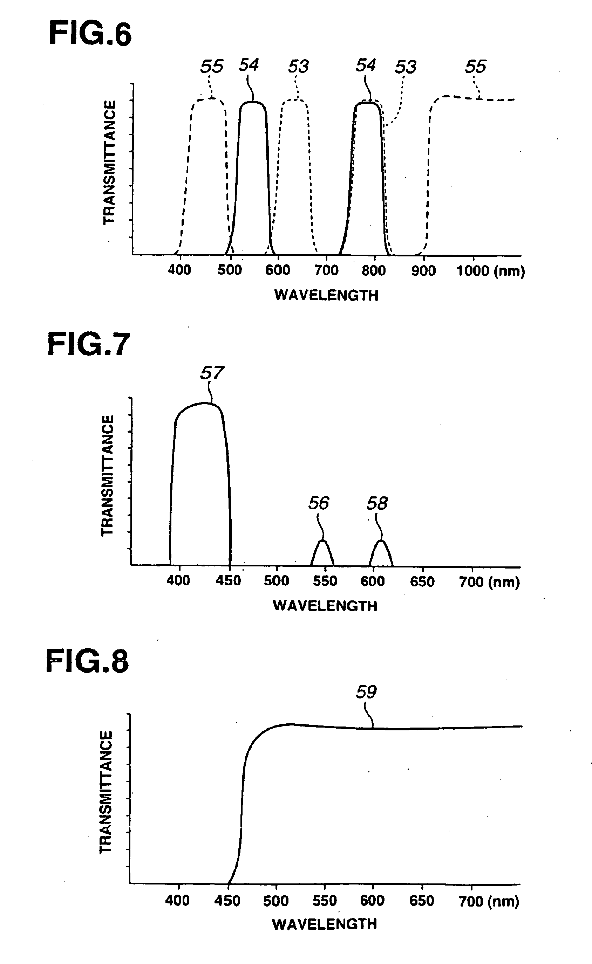

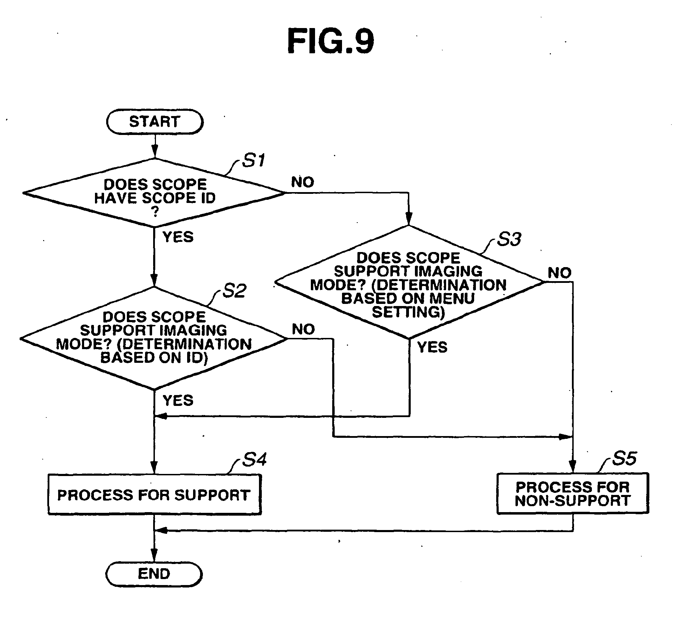

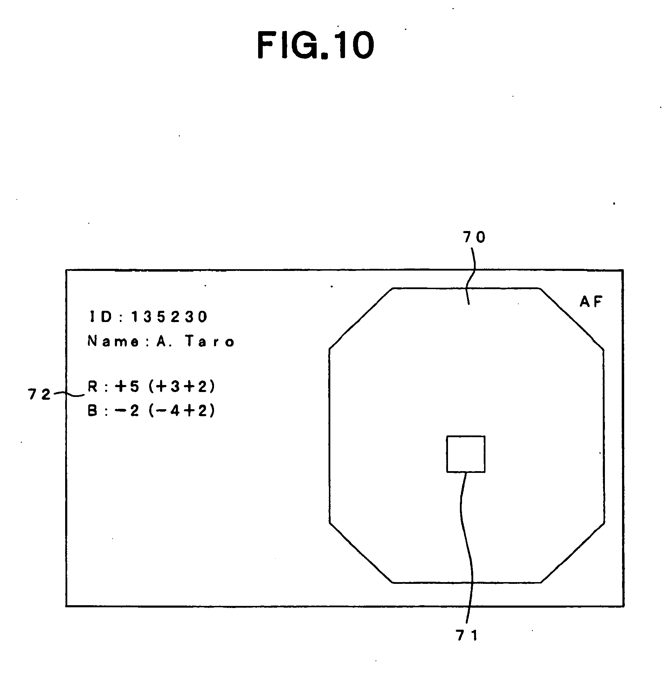

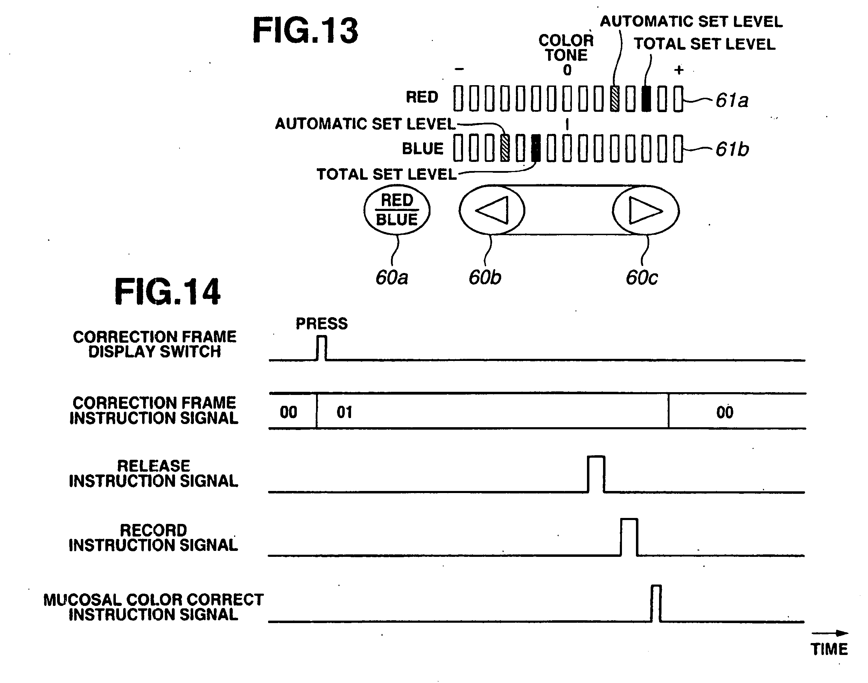

Fig. 1 is a block diagram of the entire structure of the endoscope system according to the present embodiment of the present invention, Fig. 2 is a diagram explaining a band switch filter, Fig. 3 is a diagram explaining a rotating filter wheel, Fig. 4 shows the transmission characteristic of a normal/fluorescent observation filter and that of an infrared observation filter, Fig. 5 shows the transmission characteristic of a narrow band imaging (NBI) filter, Fig. 6 shows the transmission characteristics of R, G, and B filters, Fig. 7 shows the transmission characteristics of excitation, G', and R' filters, Fig. 8 shows the transmission characteristic of an excitation-light cut filter, Fig. 9 is a flowchart of a process for determining an observation mode to be supported, Fig. 10 is a diagram explaining an observation screen, Fig. 11 is a diagram explaining the correction frame display operation, Fig. 12 explains a change in color distributions by mucosal color correction, Fig. 13 is a diagram explaining a color balance level display LED, and Fig. 14 is a diagram explaining the image recording operation.

First, the structure of the system according to the present embodiment will now be described.

Referring to Fig. 1, the endoscope system has a light source device 1, a scope 2, a processor 3, a monitor 4, a digital image recording device 5, and a keyboard 6. The light source device 1 radiates observation light to illuminate an object. The scope 2 having a charge coupled device (CCD) serving as image pickup means is inserted into a body cavity to obtain image signals. The processor 3 processes the image signals to generate image data. The monitor 4 displays an image based on the image data. The digital image recording device 5 records the image in a digital format. The keyboard 6 is connected to the processor 3. A command or characters are entered with the keyboard 6.

The light source device 1 includes a lamp 7, a band switch filter 8, a motor 9, a rotating filter wheel 10, and motors 11 and 12. The lamp 7 radiates light. The band switch filter 8 is arranged on the light path from the lamp 7 in order to control the wavelength of transmitted light. The band switch filter 8 is switched by the motor 9. The rotating filter wheel 10 is rotated by the motor 11 and is moved in the direction perpendicular to the light path by the motor 12.

The scope 2 includes a light guide fiber 15 through which illumination light passes and a long insertion portion which can be inserted into a living body. At the end of the insertion portion, an excitation-light cut filter 16 and a CCD 17 are arranged. The excitation-light cut filter 16 shuts off light having a wavelength of 460 nm or lower to intercept excitation light. The CCD 17 serves as a high-sensitive solid-state image pickup element for picking up images of light reflected by an object. An operation unit, arranged on a user's hand, includes a filter change switch 18 and a release switch 19. The user instructs to change a filter in the light source device 1 using the filter change switch 18, thus changing illumination light. The user instructs the release operation using the release switch 19. A scope ID memory 20 stores information related to the scope 2. A CPU 36 in the processor 3 can read and write information from/to the scope ID memory 20.

The present embodiment relates to the scope in which the excitation-light cut filter 16 is arranged in front of the CCD 17. In narrow band imaging, to utilize light having a wavelength ranging from 400 to 430 nm, another scope having no excitation-light cut filter is connected to the system.

The processor 3 is designed such that a supplied image signal passes through a preprocessing circuit 21, an analog to digital (A/D) conversion circuit 22, a first multiplier 23, an image processing circuit 24, a correction frame imposing circuit 25, a second multiplier 26, a gamma correction circuit 27, a selector 28, a frame memory 29, and a digital-to-analog (D/A) conversion circuit 30 in that order. An output of the A/D conversion circuit 22 is also supplied to a first sampling circuit 31. A calculated sampling value is supplied to a first coefficient control circuit 32. A coefficient, by which the corresponding image signal is multiplied, is also transmitted to the first multiplier 23. An output of the image processing circuit 24 is also supplied to a second sampling circuit 33. A calculated sampling value is supplied to a second coefficient control circuit 34. A coefficient, by which the corresponding image signal is multiplied, is transmitted to the second multiplier 26. An output of the second multiplier 26 is subjected to gamma correction through the gamma correction circuit 27 and is then supplied to the selector 28. In addition to the output of the gamma correction circuit 27, an output of the first multiplier 23 and an output of a test pattern generation circuit 35 are also supplied to the selector 28. The test pattern generation circuit 35 can generate various patterns. The CPU 36 is electrically connected to the above-mentioned internal various circuits and the external devices, i.e., the light source device 1, the scope 2, the digital image recording device 5, and the keyboard 6.

In a front panel (not shown) of the processor 3, a device variation correct switch 37, a correction frame display switch 38, a correction frame size switch 62, a mucosal color correct switch 39, a color balance set switch 60, and a color balance level display LED 61 and the like are arranged. The respective switches are electrically connected to the CPU 36. Fig. 13 shows switches and LEDs arranged in a portion of the front panel. For example, the color balance set switch 60 includes a color select switch 60a, a down switch 60b, and an up switch 60c. The color balance level display LED 61 includes a level display LED 61a for red and a level display LED 61b for blue. The LEDs 61a and 61b each have 15 elements. Each element can turn on in white or green light.

The digital image recording device 5 receives image signals generated from the processor 3 and a signal to instruct image recording, the signal being generated from the CPU 36 in the processor 3.

The keyboard 6 includes alphabetic keys to enter a patient ID or a patient name and an examination end key to delete input patient information after completion of examination.

Referring to Fig. 2, in the light source device 1, the band switch filter 8 includes a normal/fluorescent observation filter 40, a narrow band imaging (NBI) filter 41, and an infrared observation filter 42. Figs. 4 and 5 show the spectral characteristics of the respective filters.

Fig. 4 shows the characteristic 50 of the normal/fluorescent observation filter 40 and the characteristic 51 of the infrared observation filter 42. Fig. 5 shows the characteristic 52 of the NBI filter 41. The NBI filter 41 transmits light in three separate bands. Accordingly, the characteristic 52 thereof three peaks.

Referring to Fig. 3, in the light source device 1, the rotating filter wheel 10 has an R filter 43 which transmits the red (R) wavelength of light, a G filter 44 which transmits the green (G) wavelength of light, and a B filter 45 which transmits the blue (B) wavelength of light. The respective filters are arranged in the outer region of the rotating filter wheel 10. In the inner region thereof, a G' filter 46 which transmits light of 540 to 560 nm in wavelength, an excitation filter 47 which transmits excitation light of 390 to 450 nm in wavelength, and an R' filter 48 which transmits light of 600 to 620 nm in wavelength are arranged. Figs. 6 and 7 show the spectral characteristics of the respective filters in the inner and outer regions.

Fig. 6 shows the characteristics 53, 54, and 55 of the R, G, and B filters 43, 44, and 45 in the outer region of the rotating filter wheel 10. Referring to Fig. 6, the respective filters in the outer region each have the characteristic that the wavelength of near infrared light is partially transmitted in addition to that of visible light. Fig. 7 shows the respective characteristics 56, 57, and 58 of the G' filter 46, the excitation filter 47, and the R' filter 48 in the inner region.

Fig. 8 shows the characteristic 59 of the excitation-light cut filter 16 in the scope 2. The characteristic 59 does not overlap the characteristic 57 of the excitation filter 47 in Fig. 7.

Next, the operation of the endoscope system according to the present embodiment of the present invention will now be described.

The lamp 7 of the light source device 1 radiates light to illuminate an object. The light radiated from the lamp 7 is transmitted through the band switch filter 8 and the rotating filter wheel 10 and is then incident on the light guide fiber 15 of the scope 2.

In response to a filter change instruction signal from the CPU 36, the motor 9 rotates the band switch filter 8. In a normal or fluorescent observation mode, the normal/fluorescent observation filter 40 is disposed on the light path. In a narrow band imaging mode, the NBI filter 41 is arranged on the light path. In an infrared observation mode, the infrared observation filter 42 is set on the light path.

In the normal observation mode, the narrow band imaging mode, and the infrared observation mode, the rotating filter wheel 10 is arranged such that the outer filters thereof are sequentially located in the light path. The motor 11 rotates the rotating filter wheel 10 at a predetermined rotating speed, so that the R filter 43, the G filter 44, and the B filter 45 sequentially come in the light path. Due to the combination of the band switch filter 8 and the rotating filter wheel 10, the transmitted light varies. In other words, in the normal observation mode, red light, green light, and blue light are transmitted. In the narrow band imaging mode, as understood from the combination of the characteristic 52 in Fig. 5 and those in Fig. 6, light of 400 to 430 nm in wavelength, light of 530 to 560 nm, and light of 600 to 630 nm are transmitted. In the infrared observation mode, as obvious from the combination of the characteristic 51 in Fig. 4 and those in Fig. 6, light of 790 to 820 nm in wavelength and light of 900 to 980 nm are transmitted. In the fluorescent observation mode only, the motor 11 rotates at a speed that is half that in the other observation modes because an image of feeble fluorescence is picked up for a long exposure time. In the fluorescent observation mode, in response to the filter change instruction signal from the CPU 36, the motor 12 moves the rotating filter wheel 10 in the direction perpendicular to the light path, so that the inner filters are sequentially located in the light path. When the inner filter sequentially come in the light path, light of 540 to 560 nm in wavelength, light of 390 to 450 nm, and light of 600 to 620 nm are sequentially emitted from the light source device 1 according to the combination of the characteristic 50 in Fig. 4 and those in Fig. 7. In this instance, the light of 390 to 450 nm in wavelength is excitation light to excite auto-fluorescence in living-body tissue.

The light transmitted though the light guide fiber 15 of the scope 2 is then emitted from the end of the scope, thus illuminating an object such as a digestive tract. An image of light scattered, reflected, and radiated by the object is formed on the CCD 17 at the end of the scope and picked up thereby. The excitation-light cut filter 16 is arranged in front of the CCD 17, thus intercepting excitation light of 390 to 450 nm in wavelength to extract fluorescence. A CCD drive circuit (not shown) drives the CCD 17 synchronously with the rotation of the rotating filter wheel 10, so that image signals corresponding to the wavelengths of light transmitted through the R filter 43, the G filter 44, and the B filter 45 of the rotating filter wheel 10 are sequentially supplied to the processor 3.

In the processor 3, the image signals are supplied to the preprocessing circuit 21. The preprocessing circuit 21 performs processing such as correlated double sampling (CDS) to the image signals and generates the resultant image signals. The A/D conversion circuit 22 converts the signals, which are analog, generated from the preprocessing circuit 21 into digital signals. The signals generated from the A/D conversion circuit 22 are supplied to the first multiplier 23. The first multiplier 23 multiplies each image signal from the A/D conversion circuit 22 by the corresponding multiplication coefficient supplied from the first coefficient control circuit 32. The multiplication is performed using the multiplication coefficient, which is different every wavelength, synchronously with the rotation of the rotating filter wheel 10. The image processing circuit 24 performs image enhancement processing such as edge enhancement by spatial filtering.

The correction frame imposing circuit 25 imposes (namely, overlays) a frame on an image in response to an instruction from the CPU 36. The frame indicates a color sampling area serving as a reference in a process of correcting a variation caused by the color of an object, for example, a variation in mucosal color depending on patient. The process is referred to as mucosal color correction. The second multiplier 26 multiplies each signal generated from the correction frame imposing circuit 25 by the corresponding multiplication coefficient supplied from the second coefficient control circuit 34. The multiplication is performed using the multiplication coefficient, which is different every wavelength, synchronously with the rotation of the rotating filter wheel 10.

The gamma correction circuit 27 corrects the gamma characteristic of the monitor 4. The selector 28 selects any one of a signal bypassing the image processing circuit 24 and the subsequent circuits 25 to 27, a signal generated from the gamma correction circuit 27, and a signal generated from the test pattern generation circuit 35 and then generates the selected signal. Signals are written sequentially to the frame memory 29 and read simultaneously. The D/A conversion circuit 30 converts the digital signals into analog signals and then generates the signals to the monitor 4 and the digital image recording device 5.

When the scope 2 is connected to the processor 3, one observation mode (normal, auto-fluorescence, narrow band, or infrared observation mode) which the scope 2 can support, one application region (upper or lower digestive tract or a bronchus) of the scope 2, and correction parameters related to the device variation of the scope 2 are transmitted from the scope ID memory 20 to the CPU 36.

The CPU 36 determines whether the scope can support an observation mode according to a flowchart shown in Fig. 9 so that a scope having no scope ID can be connected to the processor 3.

Fig. 9 shows a flowchart to determine whether a connected scope supports an observation mode. First, whether a connected scope has a scope ID memory is determined (step S1). If the scope has the scope ID memory, whether the scope supports an observation mode is determined on the basis of data stored in the scope ID memory (step S2). If the scope supports the observation mode, a necessary process is performed (step S4). If the scope does not support the observation mode, a necessary process is performed (step S5). On the other hand, if it is determined in step S1 that the connected scope has no scope ID memory, whether the scope supports the observation mode is determined with reference to parameters set in menu setting (step S3). The menu setting includes various setting items specified by the user, e.g., whether narrow band imaging is performed (supported), and whether infrared observation is performed (supported). The user sets the items using the keyboard.

The first multiplier 23, the first sampling circuit 31, and the first coefficient control circuit 32 perform processing for correction of a color variation caused by the device variation (including a difference between models) in transmission characteristics of an optical system.

When the user corrects the color variation caused by the device variation, the user shoots an object serving as a color reference and then presses the device variation correct switch 37. Thus, the first sampling circuit 31 calculates the mean value of an image every wavelength in the sampling area. Regarding the sampling area, when the scope is connected to the system, an area suitable for the model of scope is specified by the CPU 36 and is then stored in the first sampling circuit 31. The optimum sampling area is used every model of scope. The first coefficient control circuit 32 converts the obtained mean values into a multiplication coefficient (value that is proportional to the reciprocal of the mean value) every wavelength. The multiplication coefficient is transmitted to the first multiplier 23 synchronously with the rotation of the rotating filter wheel 10. The first multiplier 23 multiplies the signal generated from the A/D conversion circuit 22 by the corresponding multiplication coefficient. Thus, the color variation caused by the device variation is suppressed in the output of the first multiplier 23. When the device variation correct switch 37 is pressed, each mean value calculated by the first coefficient control circuit 32 is stored into the scope ID memory 20 through the CPU 36. When this scope is connected another time, the mean values stored in the scope ID memory 20 are read by the first coefficient control circuit 32 through the CPU 36. Consequently, the user need not correct the device variation each time. If the connected scope 2 supports a plurality of observation modes, upon pressing the device variation correct switch 37, the device variations in the respective observation modes are automatically successively corrected.

As mentioned above, in the case where the user corrects the color variation caused by the device variation, the first multiplier 23, the first sampling circuit 31, and the first coefficient control circuit 32 constitute color balance adjustment means for adjusting the color balance of image signals, and the device variation correct switch 37 and the CPU 36 constitute control means for controlling the color balance adjustment means based on data that characterizes the color of the object.

When the user wants to easily correct a variation caused by the color of an object, e.g., a variation in mucosal color depending on patient, the user presses the correction frame display switch 38. The CPU 36 transmits a correction frame instruction signal to display a correction frame to the correction frame imposing circuit 25. Thus, as shown in Fig. 10, a correction frame 71 is displayed in an observation screen 70 of the monitor 4. The correction frame display switch 38 also functions to change the position of the correction frame 71. Each time the user presses the switch 38, the position of the frame 71 is changed to the center, the bottom, the right, the top, or the left of the observation screen 70 in that order. When the user presses the switch 38 once again, the correction frame 71 is eliminated. Fig. 11 shows the time relation between the state of the correction frame display switch 38 (pressed or not), the value of the correction frame instruction signal generated from the CPU 36, the position of the correction frame 71 displayed in the screen, and the no-frame (frame eliminating) mode. The correction frame 71 is eliminated every six presses.

When the user presses the correction frame size switch 62, the user can select any one of correction frames with different sizes. The correction frame instruction signal generated from the CPU 36 includes information related to the size of the frame. The user operates the above switches to change the position and/or size of the frame imposed by the correction frame imposing circuit 25, thus changing the position and/or size of a sampling area surrounded by the correction frame 71 where reference color sampling is performed by the second sampling circuit 33.

In the fluorescent observation mode, in order to correct a variation in color depending on patient to display a normal mucosa, which is not diseased, in a predetermined color, the user presses the mucosal color correct switch 39 while the normal mucosa is being located in the correction frame 71. When the user presses the mucosal color correct switch 39, the second sampling circuit 33 calculates the mean value of an image in an area surrounded by the correction frame 71 every wavelength. Imposing (overlaying) the correction frame 71 through the correction frame imposing circuit 25 is canceled. Assuming that Ng', Nf, and Nr' denote the respective mean values of a normal mucosal image obtained on condition that the G' filter, the excitation filter, and the R' filter are located in the light path, the second coefficient control circuit 34 selects values that are proximate to Cg', Cf, and Cr' obtained by the following expressions using Ng', Nf, and Nr' from among 15 values, i.e., $(1.2)^{-7}$, $(1.2)^{-6}$, $(1.2)^{-5}$, ..., $(1.2)^{0}$, ..., and $(1.2)^{7}$. The values (-7 to 7) of exponent parts of the selected values are referred to as automatic set levels. The automatic set levels are also transmitted to the color balance level display LED 61 through the CPU 36. A specific display example of the automatic set levels in the color balance level display LED 61 will be described below with reference to Fig. 13.

$$Cg'=1.0 \times Nf/Ng'$$

$$Cf = 1.0$$

$$Cr' = 0.5 \times Nf/Nr'$$

A multiplication coefficient corresponding to the total set level obtained by adding the automatic set level and a user set level, which will be described below, is transmitted from the second coefficient control circuit 34 to the second multiplier 26 synchronously with the rotation of the rotating filter wheel 10. Regarding the multiple coefficient, for example, when the automatic set level indicates three and the user set level indicates two, three plus two equals five, so that the total set level indicates five. Thus, the multiplication coefficient indicates $(1.2)^5$.

The second multiplier 26 multiplies the transmitted multiplication coefficient by the corresponding image signal, so that a variation caused by the color of an object, for example, a variation in mucosal color depending on patient can be corrected. In the fluorescent observation mode, when the above mucosal color correction is performed using a normal mucosa as a reference, the variation depending on patient is corrected as shown in the right of Fig. 12. Consequently, both of the color distribution of normal tissue and that of diseased tissue become smaller. A normal mucosa of any patient is displayed in light brown and a lesion is displayed in magenta on the monitor 4. Thus, the lesion is more easily discriminated from normal tissue. When the user cancels the mucosal color correction while the correction frame 71 is being displayed, the user presses the escape key in the keyboard 6. Consequently, the CPU 36 transmits an instruction to cancel the display of the correction frame 71 to the correction frame imposing circuit 25, so that imposing the correction frame 71 is canceled.

In the narrow band imaging mode and the infrared observation mode, in order to convert the color of an image into a color suitable for the corresponding observation mode, multiplication coefficients are obtained by expressions appropriate to the corresponding observation mode. Synchronously with changing the observation mode, the multiplication coefficients are changed to those corresponding to the observation mode.

As mentioned above, in the case where the user wants to easily correct a variation caused by the color of an object, e.g., a variation in mucosal color depending on patient, the second multiplier 26, the second sampling circuit 33, and the second coefficient control circuit 34 constitute color balance adjustment means for adjusting the color balance of image signals, and the mucosal color correct switch 39, the correction frame display switch 38, the correction frame size switch 62, and the CPU 36 constitute control means for controlling the color balance adjustment means based on data that characterizes the color of the object.

While the correction frame 71 is being displayed, the user can simultaneously perform color variation correction depending on patient and image recording using the release switch 19. Fig. 14 shows a time chart in the above case. When the user presses the release switch 19 while the correction frame 71 is being displayed, the release switch 19 transmits a release instruction signal to the CPU 36. The CPU 36 transmits a record instruction signal to record image data to the digital image recording device 5. After that, the CPU 36 transmits a mucosal color correct instruction signal to the second coefficient control circuit 34 in a manner similar to the operation in pressing the mucosal color correct switch 39, thus changing the multiplication coefficients. The digital image recording device 5 records the image data, which is obtained before the color is changed and in which the correction frame 71 is displayed. The recorded image data is useful when the user views the image another time and determines whether mucosal color correction has been properly performed.

When the user wants to perform fine adjustment for the color balance in order to convert the color of the image to suit their preference, the user operates the color balance set switch 60. The user selects either the red component or the blue component to be adjusted in the monitor using the color select switch 60a (see Fig. 13) and then adjusts the color tone using the down switch 60b and the up switch 60c. The level adjusted by the user is transmitted as a user set level to the second coefficient control circuit 34 through the CPU 36. In the color balance level display LED 61, the automatic set levels obtained by the mean values calculated by the second sampling circuit 33 are displayed in green (hatched portions in Fig. 13) and the total set levels obtained by adding the user set levels to the automatic set levels are displayed in white (black in Fig. 13). An image with the color tone adjusted on the basis of the combination of the total set levels is actually displayed in the monitor 4.

Fig. 13 shows the following case: Regarding the red component in the monitor 4, the automatic set level indicates +3 and the user changes the color balance by +2, so that the total set level indicates +5. For the blue component in the monitor 4, the automatic set level indicates -4 and the user changes the color balance by +2, so that the total set level indicates -2. Referring to Fig. 10, in the monitor 4, the total set level, the automatic set level, and the user set level for each of the red (R) component and the blue (B) component are displayed numerically as shown by reference numeral 72.

When the user presses the down switch 60b or the up switch 60c, the user set level, stored in a memory (not shown) in the CPU 36, of the selected color component is increased or decreased, so that the color tone of the image is changed by the respective operations of the second coefficient control circuit 34 and the second multiplier 26. In this instance, in the color balance level display LED 61, the automatic set levels are not changed but the total set level of the selected color component is changed upward or downward. In this manner, the user can arbitrarily adjust the color balance. When the user presses the mucosal color correct switch 39 to change the automatic set levels, alternatively, even when the user turns the power off, the user set levels are not changed because they are stored in a non-volatile memory externally provided for the CPU 36.

After completion of examination, when the user presses the examination end key in the keyboard 6, alternatively, when the user enters the name of the next patient, each automatic set level alone is cleared but each user set level is remained and is stored. Therefore, in the next examination, the user can observe in the color tone which suits their preference. Additionally, there is no apprehension that the user wrongly uses the automatic set levels for another patient.

As mentioned above, in the case where the user wants to perform fine color balance adjustment in order to change the color tone of an image to suit their preference, the second multiplier 26, the second sampling circuit 33, and the second coefficient control circuit 34 constitute color balance adjustment means for adjusting the color balance of image signals, and the color balance set switch 60, the color balance level display LED 61, and the CPU 36 constitute control means for controlling the color balance adjustment means on the basis of data that characterizes the color tone of an object.

For changing the observation mode, the user operates the filter change switch 18 in the scope 2 to change the mode. Each time the user presses the filter change switch 18 in the scope, the CPU 36 transmits the filter change instruction signal to the light source device 1 to change light for observation such that the mode is changed to the normal observation mode, the fluorescent observation mode, the narrow band imaging mode, or the infrared observation mode in that order. Any observation mode, which is determined that it is not supported by the connected scope on the basis of scope ID data or menu setting parameters, is skipped.

To record image data by the digital image recording device 5, the user presses the release switch 19. The release switch 19 transmits the release instruction signal to the CPU 36 and the CPU 36 then transmits the record instruction signal to the digital image recording device 5, so that the digital image recording device 5 records the image data. At that time, the CPU 36 transmits the automatic set level combination and the user set level combination related to the color balance. Data of the respective set level combinations is recorded in the header of the image data upon recording by the digital image recording device 5. In this manner, the set level combinations related to the color balance are stored in an image file. Thus, when the user reads out the image data another time, the user easily performs processing, e.g., converts the image data into image data, in which each user set level indicates zero, through a reading device and generates the converted image data.

The test pattern generation circuit 35 generates a vertically-striped gray scale chart. The selector 28 selects data related to the chart as necessary and then generates the data to the monitor 4 and the digital image recording device 5. The digital image recording device 5 records the chart. Thus, the influence of the D/A conversion circuit 30 of the processor 3 and an A/D conversion circuit of the digital image recording device 5 upon the linearity of a signal can be confirmed. Specifically, assuming that a digital value indicating 100 on the gray scale chart generated from the processor 3 is recorded as a digital value indicating 109 in the digital image recording device 5, the value recorded in the digital image recording device 5 is multiplied by about 0.9 times, thus correcting the error between DA conversion and AD conversion. Consequently, the correct value generated by the processor 3 can be obtained. When much more sampling points are actually arranged in the scale chart, the error between the devices can be more precisely corrected.

When the user wants to analyze an image, it is desirable to obtain image data which is not subjected to image processing. Image data is input to the digital image recording device 5 and is then analyzed through a personal computer. Various image analyses are known. For example, the profile of an image is examined. Image data which is not subjected to image processing such as edge enhancement is suitable for the above image analysis. In this case, the selector 28 selects a signal, which is generated from the first multiplier 23 and bypasses the image processing components, according to menu setting for the image analysis, so that image data which is not subjected to image processing and gamma correction can be generated.

In the present embodiment, the field sequential endoscope system has been described. The present invention can be applied not only to field sequential endoscope systems but also to simultaneous endoscope systems.

The first multiplier 23 can be combined with the second multiplier 26 to realize one component having two multiplying functions, thus reducing the cost.

Further, the CPU 36 can also serve as the first coefficient control circuit 32 and the second coefficient control circuit 34. This leads to the reduction in cost. The first coefficient control circuit 32, the second coefficient control circuit 34, and the CPU 36 can also be included in a field programmable gate array (FPGA).

Moreover, the sharing of the switches for various purposes leads to the cost reduction. For example, the correction frame display switch 38 or the mucosal color correct switch 39 can also be used as the color balance set switch 60.

In the above embodiment, the automatic set level combination is set in accordance with an instruction from the user. The size of the correction frame 71 may be set rather large to always update the automatic set level combination, so that the color balance can always be changed to suit an image which is being observed. In other words, in the above embodiment, only when the user generates an instruction, the automatic set level combination is updated. In this case, the user need not generate any instruction. For example, the automatic set level combination is automatically updated once per rotation of the rotating filter wheel 10 to always change the color balance to suit an image. If the size of the correction frame 71 is too small, the automatic set level combination may often fluctuate violently. Therefore, the rather large size of the correction frame 71 suppresses the violent fluctuation. The above continuous set level updating and the level setting based on an instruction of the user can also be alternatively selected.

An automatic set level combination for each patient or each region to be observed can be stored in an external device or a magnetic card, which is connected to the processor 3 and a LAN. Since the automatic set level combination is automatically read out as necessary, the user need not correct a variation in color depending on patient or region to be observed every examination.

Next, the advantages of the embodiment of the present invention will be described.

According to the present invention, since the color balance is adjusted based on data characterizing the color of an object, the color balance can be properly adjusted with simple operation, thus resulting in more precise diagnosis.

In order to accomplish the color balance adjustment with reference to a proper region in an image, a reference region can be changed. Thus, the color balance can be properly adjusted in many cases.

According to the present invention, image data is recorded by an image recording device operatively associated with the color balance adjustment. Consequently, image data serving as a reference for the color balance adjustment can be recorded with simple operation.

Further, the user can arbitrarily adjust the color balance. Observation can be performed in color suited to the user's preference. Additionally, the color balance adjustment based on data characterizing the color of an object can be performed while a display and a processing circuit are being shared. Thus, the apparatus according to the present invention can be constructed with compact circuitry.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

**Claims**

1. An endoscopic image processing apparatus that forms an image of an object having a varying color depending on a patient on an observation screen (70) of a monitor based on image signals obtained by picking up the image of the object through image pickup means (17), comprising:

   a first color balance adjustment means (23) adapted to adjust the color balance of the image signals captured by the image pickup means (17);
   a first control means (32, 36) adapted to control the first color balance adjustment means on the basis of a variation of a device coupled with the medical image processing apparatus and detected by imaging a color reference object;
   a second color balance adjustment means (26) adapted to adjust the color balance of the image signals outputted by the first color balance adjustment means (23), wherein the image signals outputted by the second color balance adjustment means (26) form the image on the observation screen (70); and
   a second control means (34, 36) adapted to control the second color balance adjustment means (26) on the basis of the color of the object having a varying color depending on a patient, **characterized in that** said color is determined within a specific region (71) of the image and said specific region is smaller than the image on the observation screen (70).

2. The apparatus according to claim 1, wherein the colour balance can be arbitrarily adjusted by a user.

3. The apparatus according to claims 1 or 2, wherein the color of the specific region of the object image picked up by the image pickup means within a correction frame (71) is determined that defines the selected area of the observation screen (70).

4. The apparatus according to claim 3, wherein the position and/or size of the specific region is changeable

5. The apparatus according to any one of claims 1 to 4, further comprising:

   storing means (5) for storing image data based on the image signals obtained by picking up the image of the object through the image pickup means (17),
   wherein a storing control means (36) instructs the storage means (5) to store the image data operatively associated with a control value for the second color balance adjustment means (26).

6. The apparatus according to any one of claims 1 to 5, wherein the image pickup means (17) is adapted to obtain image signals by applying predetermined illumination light to the object.

**Patentansprüche**

1. Endoskopbildverarbeitungsvorrichtung, die ein Bild eines Objekts mit einer sich in Abhängigkeit von einem Patienten ändernden Farbe auf einem Beobachtungsbildschirm (70) eines Monitors basierend auf Bildsignalen abbildet, die durch Aufnehmen des Bildes des Objekts durch Bildaufnahmemittel (17) erhalten werden, umfassend:

   erste Farbabgleicheinstellmittel (23), die dazu ausgebildet sind, den Farbabgleich der Bildsignale einzustellen, die durch die Bildaufnahmemittel (17) aufgenommen wurden;
   erste Steuerungsmittel (32, 36), die dazu ausgebildet sind, die ersten Farbabgleicheinstellmittel auf Grundlage einer durch Abbilden eines Farbreferenzobjektes detektierten Veränderung einer Einrichtung, die mit der medizinischen Bildverarbeitungsvorrichtung gekoppelt ist, zu steuern;
   zweite Farbabgleicheinstellmittel (26), die dazu ausgebildet sind, den Farbabgleich der Bildsignale einzustellen, die von den ersten Farbabgleicheinstellmittel (23) ausgegeben werden, wobei die von den zweiten Farbabgleicheinstellmittel (26) ausgegebenen Bildsignale das Bild auf dem Beobachtungsbildschirm (70) bilden; und
   zweite Steuerungsmittel (34, 36), die dazu ausgebildet sind, die zweiten Farbabgleicheinstellmittel (26) auf Grundlage der Farbe des Objekts mit einer in Abhängigkeit von einem Patienten ändernden Farbe zu steuern, **dadurch gekennzeichnet, dass** die Farbe innerhalb eines speziellen Bereiches (71) des Bildes bestimmt wird und der spezielle Bereich kleiner als das Bild auf dem Beobachtungsbildschirm (70) ist.

2. Vorrichtung nach Anspruch 1, wobei der Farbabgleich durch einen Nutzer willkürlich eingestellt werden kann.

3. Vorrichtung nach Anspruch 1 oder 2, wobei die Farbe des speziellen Bereichs des Bildes des Objektes, das durch die Bildaufnahmemittel aufgenommen wird, innerhalb eines Korrekturrahmens (71) bestimmt wird, der den ausgewählten Bereich des Beobachtungsbildschirms (70) definiert.

4. Vorrichtung nach Anspruch 3, wobei die Position und/oder die Größe des speziellen Bereichs veränderbar sind.

5. Vorrichtung nach einem der Ansprüche 1 bis 4, ferner aufweisend:

   Speichermittel (5) zum Speichern von Bilddaten basierend auf den Bildsignalen, die durch Aufnehmen des Bildes des Objekts durch die Bildaufnahmemittel (17) erhalten werden, wobei Speichersteuerungsmittel (36) die Speichermittel (5) so anweisen, dass die Bilddaten einem Steuerungswert für die zweiten Farbabgleicheinstellmittel (26) betriebsfähig zugeordnet gespeichert werden.

6. Vorrichtung nach einem der Ansprüche 1 bis 5, wobei die Bildaufnahmemittel (17) dazu ausgebildet sind, Bildsignale durch Anwenden eines vorbestimmten Beleuchtungslichts auf das Objekt zu erhalten.

**Revendications**

1. Appareil de traitement d'images endoscopiques qui forme une image d'un objet ayant une couleur variable en fonction d'un patient sur un écran d'observation (70) d'un moniteur sur la base de signaux d'image obtenus en captant l'image de l'objet par l'intermédiaire d'un moyen de capteur d'images (17), comprenant :

   un premier moyen d'ajustement de l'équilibre des couleurs (23) adapté pour ajuster l'équilibre des couleurs des signaux d'image capturés par le moyen de capteur d'images (17) ;
   un premier moyen de commande (32, 36) adapté pour commander le premier moyen d'ajustement de l'équilibre des couleurs sur la base d'une variation d'un dispositif couplé avec l'appareil de traitement d'images médical et détectée en imageant un objet de référence en couleur ;
   un deuxième moyen d'ajustement de l'équilibre des couleurs (26) adapté pour ajuster l'équilibre des couleurs des signaux d'image délivrés en sortie par le premier moyen d'ajustement de l'équilibre des couleurs (23), dans lequel les signaux d'image délivrés en sortie par le deuxième moyen d'ajustement de l'équilibre des couleurs (26) forment l'image sur l'écran d'observation (70) ; et
   un deuxième moyen de commande (34, 36) adapté pour commander le deuxième moyen d'ajustement de l'équilibre des couleurs (26) sur la base de la couleur de l'objet ayant une couleur variable en fonction d'un patient, **caractérisé en ce que** ladite couleur est déterminée à l'intérieur d'une région spécifique (71) de l'image et ladite région spécifique est plus petite que l'image sur l'écran d'observation (70).

2. Appareil selon la revendication 1, dans lequel l'équilibre des couleurs peut être ajusté arbitrairement par un utilisateur.

3. Appareil selon les revendications 1 ou 2, dans lequel la couleur de la région spécifique de l'image de l'objet captée par le moyen de capteur d'images à l'intérieur d'un cadre de correction (71) est déterminée qui définit la zone sélectionnée de l'écran d'observation (70).

4. Appareil selon la revendication 3, dans lequel la position et/ou la taille de la région spécifique est changeable.

5. Appareil selon l'une quelconque des revendications 1 à 4, comprenant en outre :

un moyen de stockage (5) pour stocker des données d'images sur la base des signaux d'image obtenus en captant l'image de l'objet par l'intermédiaire du moyen de capteur d'images (17), dans lequel un moyen de commande de stockage (36) ordonne au moyen de stockage (5) de stocker les données d'images opérationnellement associées avec une valeur de commande pour le deuxième moyen d'ajustement de l'équilibre des couleurs (26).

6. Appareil selon l'une quelconque des revendications 1 à 5, dans lequel le moyen de capteur d'images (17) est adapté pour obtenir des signaux d'images en appliquant une lumière d'éclairage prédéterminée à l'objet.

EP 1 527 729 B1

# FIG.1

FIG.1 block diagram showing: PREPROCESSING (21), A/D (22), IMAGE PROCESSING (24), CORRECTION FRAME IMPOSING (25), GAMMA (27), SELECTOR (28), FRAME MEMORY (29), D/A (30), MONITOR (4), DIGITAL IMAGE RECORDING (5), KEYBOARD (6), multiplier (23), SAMPLING (31), COEFFICIENT CONTROL (32), multiplier (26), TEST PATTERN (35), SAMPLING (33), COEFFICIENT CONTROL (34), CPU (36), DEVICE VARIATION CORRECT SWITCH (37), CORRECTION FRAME DISPLAY SWITCH (38), CORRECTION FRAME SIZE SWITCH (62), MUCOSAL COLOR CORRECT SWITCH (39), COLOR BALANCE SET SWITCH (60), COLOR BALANCE LEVEL DISPLAY LED (61), CORRECTION FRAME INSTRUCTION, USER SET LEVEL/MUCOSAL COLOR CORRECT INSTRUCTION, AUTOMATIC SET LEVEL, RECORD INSTRUCTION, FILTER CHANGE INSTRUCTION, FILTER CHANGE SWITCH (18), RELEASE SWITCH (19), SCOPE ID MEMORY (20), 3 PROCESSOR, CCD (17), EXCITATION-LIGHT CUT FILTER (16), 2 SCOPE, 15 LIGHT GUIDE FIBER, ROTATING FILTER WHEEL (10), MOTOR (11), MOTOR (12), BAND-PASS CHANGE FILTER (8), 9 MOTOR, LAMP (7), 1 LIGHT SOURCE DEVICE, SUBJECT.

12

# FIG.2

# FIG.3

# FIG.4

# FIG.5

# FIG.6

# FIG.7

# FIG.8

# FIG.9

```
        ┌─────────┐
        │  START  │
        └─────────┘
             │
             ▼
          S1 ╱
      ╱─────────────╲          NO
     ╱ DOES SCOPE    ╲──────────────┐
     ╲ HAVE SCOPE ID ╱              │
      ╲      ?      ╱               │
       ╲───────────╱                ▼
             │                   S3 ╱
            YES            ╱──────────────────╲     NO
             │           ╱  DOES SCOPE         ╲─────────┐
             ▼          ╱  SUPPORT IMAGING      ╲         │
          S2 ╱         ╲  MODE? (DETERMINATION  ╱         │
     ╱───────────────╲  ╲  BASED ON MENU        ╱         │
    ╱  DOES SCOPE     ╲  ╲    SETTING)         ╱          │
   ╱  SUPPORT IMAGING  ╲  ╲──────────────────╱           │
   ╲  MODE? (DETERMINATION NO    │                       │
    ╲  BASED ON ID)    ╱────┐   YES                      │
     ╲───────────────╱      │    │                       │
             │              └────┤                       │
            YES                  │                       │
             │         ┌─────────┘                       │
             │         │                                 │
             ▼         ▼                                 ▼
          S4 ╱                              S5 ╱
    ┌──────────────────────┐        ┌──────────────────┐
    │ PROCESS FOR SUPPORT  │        │ PROCESS FOR      │
    │                      │        │ NON-SUPPORT      │
    └──────────────────────┘        └──────────────────┘
             │                                 │
             ▼                                 │
          ┌────────┐◄───────────────────────────
          │  END   │
          └────────┘
```

# FIG.10

70

AF

ID : 1 3 5 2 3 0
Name : A. Taro

72 R : +5 (+3+2)
B : −2 (−4+2)

71

# FIG.11

| CORRECTION FRAME DISPLAY SWITCH | PRESS | PRESS | PRESS | PRESS | PRESS | PRESS | PRESS | PRESS | PRESS |
|---|---|---|---|---|---|---|---|---|---|

| CORRECTION FRAME INSTRUCTION SIGNAL | 00 | 01 | 02 | 03 | 04 | 05 | 00 | 01 | 02 | 03 |
|---|---|---|---|---|---|---|---|---|---|---|

| CORRECTION FRAME POSITION | NOTHING | CENTER | BOTTOM | RIGHT | TOP | LEFT | NOTHING | CENTER | BOTTOM | RIGHT |
|---|---|---|---|---|---|---|---|---|---|---|

TIME

# FIG.12

FLUORESCENCE INTENSITY

DISTRIBUTION OF NORMAL TISSUE

DISTRIBUTION OF DISEASED TISSUE

R' REFLECTED LIGHT INTENSITY

G' REFLECTED LIGHT INTENSITY

MUCOSAL COLOR CORRECTION

FLUORESCENCE INTENSITY

DISTRIBUTION OF NORMAL TISSUE

DISTRIBUTION OF DISEASED TISSUE

R' REFLECTED LIGHT INTENSITY

G' REFLECTED LIGHT INTENSITY

EP 1 527 729 B1

# FIG.13

COLOR TONE

AUTOMATIC SET LEVEL

TOTAL SET LEVEL

− 0 +

RED 〔〕〔〕〔〕〔〕〔〕〔〕〔〕〔〕〔〕▨〔〕█〔〕〔〕 ～61a

AUTOMATIC SET LEVEL

BLUE 〔〕〔〕〔〕▨〔〕█〔〕〔〕〔〕〔〕〔〕〔〕〔〕〔〕 ～61b

TOTAL SET LEVEL

$\dfrac{\text{RED}}{\text{BLUE}}$   ◁   ▷

60a      60b        60c

# FIG.14

| CORRECTION FRAME DISPLAY SWITCH | PRESS |
|---|---|

| CORRECTION FRAME INSTRUCTION SIGNAL | 00 | 01 | 00 |

RELEASE INSTRUCTION SIGNAL

RECORD INSTRUCTION SIGNAL

MUCOSAL COLOR CORRECT INSTRUCTION SIGNAL

TIME

**EP 1 527 729 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002336196 A
- JP 2002095635 A
- US 5570129 A
- US 5926213 A
- US 6421083 B
- US 6293911 B
- US 5058603 A
- WO 0126050 A